# DEMANDE DE BREVET EUROPEEN

(11) **EP 4 248 942 A1**
(43) Date de publication de la demande: **27.09.2023**
(21) Numéro de dépôt: 23163908.9
(22) Date de dépôt: 24.03.2023
(51) Int. Cl.: A61K 8/60, A61K 8/99, A61Q 19/02, A61Q 19/08

(54) **EXTRAIT DE SPIRODELA POLYRHIZA ET SES UTILISATIONS COSMÉTIQUES**

(30) Priorité: 25.03.2022 FR 2202694
(71) Demandeur: Societe Industrielle Limousine d'Application Biologique, 19130 Objat (FR)
(72) Inventeur: PAUFIQUE, Jean, 19130 OBJAT (FR)
(74) Mandataire: Aquinov

(57) **Abrégé**

La présente invention concerne un principe actif cosmétique obtenu à partir d'une lentille d'eau de l'espèce *Spirodela polyrhiza* et ses utilisations cosmétiques, en particulier son utilisation comme repulpant.

## Description

### Domaine technique

L'invention concerne un principe actif cosmétique obtenu à partir d'une lentille d'eau de l'espèce *Spirodela polyrhiza* et ses utilisations cosmétiques, en particulier son utilisation comme repulpant.

### Etat de l'art

L'industrie cosmétique porte un intérêt certain pour les problématiques liées à la déshydratation, en particulier la déshydratation cutanée. Les peaux déshydratées sont caractérisées par un déficit en eau dont les causes sont multifactorielles. Parmi les facteurs les plus fréquents, on peut notamment citer les facteurs environnementaux, hormonaux, ou encore ceux liés à la prise de médicaments. Selon une idée reçue, une peau déshydratée est une peau sèche. Toutefois, tous les types de peau peuvent être touchés, que la peau soit sèche, mixte ou grasse. La sensation de tiraillements, de manque de souplesse, de perte de volume, d'un teint terne, de rides et ridules qui apparaissent sont autant de manifestations qui sont symptomatiques des peaux déshydratées.

Il existe donc un besoin pour de nouveaux principes actifs cosmétiques présentant un haut potentiel hygroscopique permettant l'effet repulpant, l'hydratation et l'éclat du teint de la peau.

Pour répondre à ce besoin, l'inventeur s'est intéressé à des matières premières naturelles pour développer des ingrédients présentant un potentiel hygroscopique élevé. Un intérêt particulier a été porté aux macromolécules de type pectines issues du monde aquatique et spécifiquement à celles issues de la lentille d'eau.

Utilisée depuis plus de 2 000 ans en médecine traditionnelle chinoise pour ses capacités à promouvoir le métabolisme de l'eau, la lentille d'eau présente une paroi végétale comprenant des pectines originales, contribuant ainsi à retenir l'eau dans les cellules végétales de la plante. Ces pectines de structure originale, les apiogalacturonanes (APG) ont été identifiées uniquement chez quelques espèces aquatiques, comme dans les espèces *Lemna minor, Zostera marina, qui* sont par ailleurs connues en cosmétique pour leurs effets antioxydants.

Dans ce contexte, l'inventeur s'est intéressé à une espèce de lentille d'eau particulière, l'espèce *Spirodela polyrhiza,* et a découvert son potentiel hygroscopique supérieur à d'autres glycocomposés d'origine pectique (homogalacturonanes) ou non pectique (fructosanes) et ses remarquables propriétés hydratantes et repulpantes.

### Résumé de l'invention

Ainsi, l'invention concerne un nouveau principe actif cosmétique obtenu à partir d'une lentille d'eau appartenant à l'espèce *Spirodela polyrhiza* comprenant des apiogalacturonanes, en particulier son utilisation comme repulpant. Préférentiellement, le principe actif selon l'invention est enrichi en apiogalacturonanes.

La lentille d'eau *Spirodela polyrhiza* également dénommée *Lemna polyrhiza,* ou *Lemna major* ou *Spirodela polyrrhiza* appartenant à la famille des *Lemnacées,* est une plante aquatique vivace recouvrant la surface des étangs par flottaison permettant ainsi de réguler les variations de température entre l'air et l'eau. Elle est constituée d'une fronde (ou thalle) pouvant atteindre 10mm de diamètre, remplie de minuscules poches d'air lui permettant de flotter, présentant souvent de 5 à 12 nervures, et comprenant de 2 à 16 racines fasciculées ne dépassant pas 3 cm de long.

La lentille d'eau *Spirodela polyrhiza* est dotée d'une paroi pectique originale contenant des apiogalacturonanes (APG). Leur rôle est d'assurer les échanges hydriques ainsi que de conférer une grande plasticité à la plante lors de sa multiplication. Sous leur forme native, les apiogalacturonanes de *Spirodela polyrhiza* présentent une structure linéaire composée d'une chaîne linéaire d'acides galacturoniques formée par des liaisons fortes, et des ramifications de motifs di-apioses branchées par des liaisons faibles, extrêmement fragiles. Des effets dépolluants, antioxydants, dépigmentants, anti-rides, stimulant la pousse des cheveux, cils ou sourcils ont précédemment été rapportés pour des extraits de lentilles d'eau.

Aussi, l'invention se rapporte à un principe actif comprenant au moins un extrait de *Spirodela polyrhiza* riche en apiogalacturonanes, ainsi qu'à son utilisation cosmétique préférentiellement comme repulpant et/ou hydratant et/ou pour améliorer l'éclat du teint.

Afin de développer un tel ingrédient dérivé du naturel (selon les définitions de la norme ISO 16128) enrichi en apiogalacturonanes tout en préservant leurs structures natives, notamment les motifs di-apioses, l'extrait compris dans le principe actif selon l'invention est préférentiellement un hydrolysat. Celui-ci peut être réalisé par une hydrolyse chimique, ou par une hydrolyse chimique et une hydrolyse enzymatique.

Avantageusement, le principe actif selon l'invention présente un potentiel hygroscopique supérieur ou égal à 15 molécules d'eau en interaction par monomère.

L'extrait selon l'invention a été caractérisé et celui-ci comprend des carbohydrates, communément appelés sucres. Préférentiellement, ceux-ci représentent au moins 70% en poids de la matière sèche de l'extrait.

Les carbohydrates compris dans l'extrait selon l'invention comprennent particulièrement des apiogalacturonanes, qui représentent préférentiellement au moins 40% en poids de la matière sèche de l'extrait, plus préférentiellement entre 40 et 70% en poids de la matière sèche de l'extrait.

Aussi, l'extrait selon l'invention comprend des carbohydrates composés d'apiogalacturonanes et éventuellement d'autres sucres. Ces autres sucres représentent préférentiellement entre 20 et 35% en poids de la matière sèche de l'extrait. A titre d'exemple, les autres sucres peuvent être issus des rhamnogalacturonanes-I (RG-I).

Les apiogalacturonanes présents dans l'extrait sont composés d'une chaîne linéaire d'acides galacturoniques branchée de motifs apioses, plus préférentiellement de motifs di-apioses. L'extrait selon l'invention comprend préférentiellement des apioses liés.

Selon un objet préféré de l'invention, l'extrait présente un ratio acide galacturonique / apiose compris entre 1 et 5, préférentiellement entre 2 et 4.

Selon un autre objet, les apiogalacturonanes présentent une masse molaire comprise entre 180Da et 1360 kDa, ainsi qu'une masse molaire moyenne de 38 kDa. Préférentiellement au moins 40% des apiogalacturonanes présentent une masse molaire comprise entre 3600 Da et 200 kDa.

Selon un objet particulièrement préféré, l'extrait est susceptible d'être obtenu par un procédé comprenant les étapes suivantes :
a. Solubilisation d'au moins 50 g/L de *Spirodela polyrhiza* dans l'eau,
b. Hydrolyse chimique de nature acide, ou hydrolyse chimique de nature acide et hydrolyse enzymatique,
c. Séparation des phases solubles et insolubles et récupération de la phase soluble,
d. Traitement thermique
e. Purification et concentration de la phase soluble,
f. Filtration et filtration stérilisante.

L'invention se rapporte également à un procédé de préparation du principe actif selon l'invention, comprenant notamment une solubilisation d'une biomasse de *Spirodela polyrhiza* dans l'eau, une hydrolyse chimique de nature acide, ou une hydrolyse chimique et une hydrolyse enzymatique, une séparation de la phase soluble et insoluble, puis récupération de la phase soluble, une purification et éventuellement une concentration de la phase soluble suivie d'une filtration et filtration stérilisante.

Le principe actif selon l'invention présente avantageusement un effet choisi parmi un effet repulpant, booster d'éclat c'est-à-dire améliorant l'éclat du teint, et hydratant, permettant ainsi de répondre aux problématiques de l'art antérieur. Aussi, il peut être utilisé pour des applications cosmétiques et être ainsi intégré dans une composition cosmétique, avantageusement une composition sous une forme adaptée à une application topique.

Ainsi, selon un autre aspect, l'invention se rapporte à une composition cosmétique pour une application topique comprenant au moins un principe actif selon l'un des quelconques modes de réalisation précédemment décrits et un milieu physiologiquement acceptable.

Préférentiellement, le principe actif contenu dans la composition selon l'invention représente au moins 0,1% en poids du poids total de la composition.

Le principe actif ou la composition selon l'invention est destiné à des applications cosmétiques. Ainsi, selon un autre aspect, l'invention concerne l'utilisation cosmétique du principe actif selon l'un des quelconques modes de réalisation ou d'une composition selon l'un des quelconques modes de réalisation. Préférentiellement l'utilisation cosmétique vise une application topique permettant d'obtenir un effet repulpant et/ou un hydratant et/ou améliorant l'éclat du teint.

En particulier, le principe actif ou la composition selon l'invention augmente le volume cellulaire cutané, préférentiellement le volume des kératinocytes, et/ou l'expression de l'aquaporine 3 et/ou la synthèse du transporteur BGT-1.

Enfin, selon un dernier aspect, l'invention a également pour objet un procédé de traitement cosmétique des peaux déshydratées qui consiste en l'application sur la peau déshydratée d'une composition selon l'invention ou d'un principe actif tel que décrit précédemment.

D'autres caractéristiques et avantages ressortiront de la description détaillée de l'invention, des exemples et des figures qui vont suivre.

### Brève description des Figures

[Fig. 1] représente l'effet du principe actif selon l'invention, des extraits hors invention selon les Exemples 3 et 4, sur la quantité, la profondeur et le maintien dans le temps du l'eau D2O captée par la peau, 3 heures après une application unique.

[Fig. 2] représente l'effet du principe actif selon l'invention sur la quantité d'eau totale et la profondeur au niveau de l'épiderme de volontaires 3h après une application unique.

[Fig. 3] représente l'effet du principe actif selon l'invention sur la quantité d'eau totale au niveau de l'épiderme de volontaires caucasiens après 42 jours d'application biquotidienne.

[Fig. 4] représente l'effet du principe actif selon l'invention sur l'éclat du teint après 21 et 42 jours de traitement biquotidien par des volontaires caucasiens.

[Fig. 5] représente l'effet du principe actif selon l'invention sur l'éclat du teint après 21 et 42 jours de traitement biquotidien par des volontaires asiatiques.

### Description détaillée de l'invention

### Définition

Par « principe actif cosmétique » au sens de l'invention, on entend un extrait comprenant au moins une molécule, préférentiellement un ensemble de molécules présentant un effet cosmétique sur la peau, lorsqu'il est appliqué topiquement.

Par « ingrédient dérivé du naturel » au sens de l'invention, on entend un ingrédient cosmétique obtenu par des procédés chimiques et/ou biologiques définis visant à le modifier chimiquement. Ces modifications chimiques sont intentionnelles, et les structures des molécules obtenues ne sont donc plus identiques aux molécules présentes dans la nature.

Par « hydrolysat de *Spirodela polyrhiza* » au sens de l'invention, on entend un principe actif issu de la lentille d'eau de l'espèce *Spirodela polyrhiza,* obtenu par un procédé comprenant au moins une étape d'hydrolyse de *Spirodela polyrhiza.* Le terme hydrolysat de *Spirodela polyrhiza* exclut les molécules produites uniquement par fermentation de *Spirodela polyrhiza* par un micro-organisme ou une infusion/décoction/macération de *Spirodela polyrhiza.*

Par « *Spirodela polyrhiza* » au sens de l'invention, on entend la lentille d'eau de la famille des *Lemnacées* et de l'espèce *Spirodela polyrhiza* également connu sous les noms *Lemna polyrrhiza, Lemna major, Lemna maxima, Spirodela polyrrhiza,* excluant ainsi les espèces *Lemna minor* ou *Zostera marina.*

Par « repulpant » au sens de l'invention, on entend un effet permettant d'augmenter le volume cellulaire cutané. Cet effet est souvent associé au terme « peau rebondie ».

Par « potentiel hygroscopique d'une molécule » au sens de l'invention, on entend la capacité de la structure d'une molécule à interagir, à lier et à retenir des molécules d'eau autour de sa structure chimique.

Par « peaux déshydratées » au sens de l'invention, on entend une peau souffrant d'un déficit en eau, présentant ainsi une sensation d'inconfort, de tiraillement, d'un manque de souplesse. Ces signes de déshydratation ne sont pas ceux d'une peau sèche qui souffre d'un manque de matière grasse. Une peau déshydratée touche tous les types de peau qu'elle soit sèche, mixte ou grasse.

Par « monomère » au sens de l'invention, on entend un monosaccharide d'acide galacturonique ou un monosaccharide d'apiose

Par « ratio d'acide galacturonique / apiose » au sens de l'invention, on entend le ratio entre le nombre de mole d'acide galacturonique et le nombre de mole d'apiose dans les apiogalacturonanes.

### Principe actif selon l'invention

La présente invention a donc pour objet un principe actif cosmétique comprenant au moins un extrait de *Spirodela polyrhiza,* l'extrait comprenant des apiogalacturonanes. Un tel principe actif est particulièrement d'intérêt et permet de répondre aux inconvénients de l'art antérieur en ce qu'il présente un potentiel hygroscopique élevé obtenu par la préservation des molécules d'apiogalacturonanes compris dans l'extrait de *Spirodela polyrhiza.* Grâce à ses propriétés hygroscopiques remarquables, le principe actif selon l'invention hydrate la peau du *stratum corneum* jusqu'au derme supérieur se traduisant par un effet repulpant, hydratant ou booster d'éclat.

Aussi, le principe actif selon l'invention présente préférentiellement un potentiel hygroscopique supérieur ou égal à 15 molécules d'eau en interaction par monomère, plus préférentiellement supérieur ou égal à 20 molécules d'eau en interaction par monomère.

Le principe actif selon l'invention est enrichi en apiogalacturonanes, préférentiellement les apiogalacturonanes représentent au moins 40% en poids de la matière sèche de l'extrait, plus préférentiellement ils représentent entre 40% et 70% en poids de la matière sèche de l'extrait.

Le principe actif selon l'invention comprend des sucres, en particulier des carbohydrates (sucres neutres et acides uroniques), les carbohydrates représentent préférentiellement au moins 70% en poids de la matière sèche de l'extrait. La teneur en sucres neutres totaux dans ledit principe actif peut être déterminée par la méthode de DUBOIS (Dubois M. et al., Analytical chemistry, 28, 3, 350-356, 1956). La teneur en sucres neutres totaux dans le principe actif selon l'invention est exprimée en pourcentage par rapport à la matière sèche.

Les carbohydrates présents dans l'extrait peuvent être sous forme majoritairement de polysaccharides, d'oligosaccharides et très peu de monosaccharides. Ainsi, les carbohydrates du principe actif selon l'invention ont une masse molaire moyenne de 38kDa, et des masses molaires comprises entre 180Da et 1 360 kDa.

En particulier, les carbohydrates présents dans l'extrait selon l'invention comprennent des apiogalacturonanes et d'autres sucres. Préférentiellement, les autres sucres représentent entre 20 et 35% en poids de la matière sèche de l'extrait. Les autres sucres peuvent par exemple comprendre du rhamnose, du galactose, issus du rhamnogalacturonanes-I (RG-I), de l'arabinose ou du glucose.

Les apiogalacturonanes présents dans l'extrait sont préférentiellement composés d'une chaîne linéaire d'acides galacturoniques branchée de ramifications de motifs di-apioses.

Le principe actif cosmétique selon l'invention peut se présenter sous forme liquide, sous forme solide ou sous forme de film.

Lorsqu'il se présente sous forme liquide, le principe actif selon l'invention est exclusivement constitué par l'extrait de *Spirodela polyrhiza* accompagné de stabilisant et/ou système de conservation.

L'extrait sous forme liquide se présente préférentiellement sous forme d'une solution aqueuse liquide limpide, avec une faible odeur et une couleur jaune très pâle à jaune clair. Il peut toutefois être plus coloré et/ou être décoloré par tout procédé connu de l'homme du métier.

L'inventeur a également déterminé la teneur en matières sèches de l'extrait. Celle-ci peut être déterminée par la pesée des résidus issus du séchage de l'extrait selon l'invention à 105°C dans une étuve jusqu'à l'obtention d'un poids constant. Préférentiellement, l'extrait selon l'invention sous forme liquide a une teneur en matières sèches de 3g/l à 20g/l, préférentiellement de 5 g/L à 8 g/L.

Lorsqu'il se présente sous forme solide, le principe actif selon l'invention est préférentiellement constitué par l'extrait de *Spirodela polyrhiza* tel que précédemment décrit et par un support choisi parmi la maltodextrine, la gomme arabique, la lécithine de soja ou l'isomalt. Selon un mode de réalisation, particulièrement adapté, l'extrait représente au moins 10% en poids du principe actif et le support au plus 90% en poids du principe actif.

Dans le cas d'une forme solide dans laquelle le principe actif est associé à un support, les teneurs en protéines, en sucres et en cendres dans le principe actif sont modifiées, le support étant généralement constitué majoritairement de sucres.

Le principe actif selon l'invention peut aussi être présenté sous forme d'un film. Dans ce cas, l'extrait de *Spirodela polyrhiza* représente préférentiellement au moins 0,1% en poids du film.

Lorsqu'il se présente sous forme de film, le principe actif comprend :
- au moins l'extrait de *Spirodela polyrhiza* selon l'invention.
- au moins une charge minérale, et
- au moins un polymère d'origine naturelle, et
- au moins un plastifiant, et
- au moins un tensio-actif, et

Le polymère d'origine naturelle peut être choisi parmi la : Pectine, Gomme tamarin, Alginate, Pullulane, Psyllium, Xanthane, Guar, Tara, Caroube, Agar, Gomme arabique, Gellane, Dextran, Carraghénane, Cellulose, Konjac et le Chitosan.

Le plastifiant peut être choisi parmi : Glycérol, Sorbitol, Saccharose, Erythritol, Urée, Propylène glycol et le Butylene glycol.

La charge minérale peut être choisie parmi : Carbonate de calcium, argile verte, kaolin, perlite, talc, silicate de magnésium, mica, sericite diatomée, silice, sulfate de calcium chlorure de calcium, chlorure de potassium, oxyde de fer et l'oxyde de zinc.

Le principe actif peut comprendre en outre un pigment pour colorer le film.

L'extrait de *Spirodela polyrhiza* est préférentiellement un hydrolysat. L'hydrolysat peut être obtenu par tous types d'hydrolyse, préférentiellement par une hydrolyse chimique de nature acide, ou par une hydrolyse chimique de nature acide et une hydrolyse enzymatique.

Aussi, l'extrait selon l'invention est préférentiellement un hydrolysat acide ou un hydrolysat acido-enzymatique.

Selon un mode de réalisation particulièrement préféré, l'extrait du principe actif cosmétique selon l'invention est susceptible d'être obtenu par un procédé d'extraction comprenant les étapes suivantes :
a. Solubilisation d'au moins 50 g/L de *Spirodela polyrhiza* dans l'eau,
b. Hydrolyse chimique de nature acide, ou hydrolyse chimique et hydrolyse enzymatique,
c. Séparation des phases solubles et insolubles et récupération de la phase soluble,
d. Traitement thermique,
e. Purification et concentration de la phase soluble,
f. Filtration et filtration stérilisante.

### Composition cosmétique selon l'invention

Le principe actif selon l'invention peut éventuellement être intégré dans une composition cosmétique, notamment une composition comprenant au moins 0,1% en poids dudit principe actif et un milieu physiologiquement acceptable, préférentiellement un milieu cosmétiquement acceptable.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux, poudres, ou fond de teint ou sous forme de film. Elles peuvent être plus ou moins fluides et avoir l'aspect de crèmes, émulsions, gels, masques ou tous autres aspects des cosmétiques de soin de la peau saine.

Il peut s'agir de compositions comprenant au moins 0,1% du principe actif liquide selon l'invention, préférentiellement entre 0,1 et 10%.

Ces compositions comprennent, outre le principe actif, un milieu physiologiquement acceptable tel qu'un milieu cosmétiquement acceptable, c'est-à-dire qui ne provoque pas de sensations d'inconfort pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles,
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (International Cosmetic ingrédient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

### Procédé d'extraction de l'extrait selon l'invention

L'extrait constituant ou contenu dans le principe actif selon l'invention peut être obtenu par tout moyen.

Selon un mode de réalisation préféré, le procédé d'extraction comprend au moins une étape d'extraction de la lentille d'eau *Spirodela polyrhiza,* tel qu'une étape d'hydrolyse choisie parmi une hydrolyse chimique de nature acide, une hydrolyse enzymatique et leur combinaison.

Préalablement au procédé d'obtention de l'extrait en tant que tel, une culture de la biomasse de *Spirodela polyrhiza* est réalisée dans un milieu adapté à leur développement, par exemple un milieu de culture adapté, de manière classique pour l'homme de métier. Une fois la biomasse obtenue, on réalise une étape d'extraction, préférentiellement une hydrolyse acide, ou des hydrolyses acide et enzymatique en vue d'obtenir les molécules actives.

Selon un mode de réalisation particulièrement adapté, le principe actif selon l'invention est obtenu par la mise en oeuvre des étapes suivantes :
a. Solubilisation d'au moins 50 g/L de *Spirodela polyrhiza* dans l'eau,
b. Hydrolyse chimique de nature acide, ou hydrolyse chimique de nature acide et hydrolyse enzymatique,
c. Séparation des phases solubles et insolubles et récupération de la phase soluble,
d. Purification et concentration de la phase soluble,
e. Filtration et filtration stérilisante.

Les conditions d'hydrolyse sont choisies pour obtenir un extrait enrichi en apiogalacturonanes préservant une structure d'acides galacturoniques et des ramifications di-apioses.

La séparation de la phase soluble et insoluble est réalisée par tout moyen connu de l'homme du métier, par exemple par centrifugation, filtration ou décantation. La séparation des phases soluble et insoluble est réalisée pour récupérer la phase soluble contenant entre autres les sucres solubles, tels que les apiogalacturonanes.

Eventuellement, une étape supplémentaire de traitement thermique peut être réalisée pour inactiver l'enzyme utilisée. Cette inactivation est alors réalisée selon les préconisations du fournisseur de l'enzyme utilisée.

Eventuellement, le procédé comprend une étape de filtration après la récupération de la phase soluble pour éliminer les particules encore en suspension. Ainsi, cette étape de filtration permet la purification de la phase soluble récupérée et est réalisée afin sélectionner les molécules actives.

L'hydrolysat obtenu à ce stade peut éventuellement être encore concentré et/ou purifié, préférentiellement par des étapes d'ultrafiltration successives à travers des membranes de porosité différentes, en conservant les molécules actives à chaque étape et/ou par une méthode de type chromatographique.

L'hydrolysat obtenu après hydrolyse et filtration, avant ou après concentration et filtration stérilisante, est un hydrolysat de *Spirodela polyrhiza,* et constitue une première forme du principe actif selon l'invention, se présentant alors sous forme liquide.

L'hydrolysat obtenu peut ensuite être séché et associé ou non à un support, pour se présenter sous forme solide. Cette phase peut être réalisée par la mise en oeuvre des étapes suivantes :
- un support d'atomisation, de préférence la maltodextrine, est ajouté dans l'hydrolysat de *Spirodela polyrhiza,* d'au plus 90% (en masse/volume) ;
- cette solution est ensuite concentrée sous vide ;
- une élimination des bactéries éventuellement présentes est réalisée par traitement thermique ;
- l'atomisation permet d'obtenir une poudre.

Les étapes des procédés décrits ci-avant, prises individuellement sont usuelles dans le domaine des extractions d'actifs à partir de matières premières naturelles et l'homme du métier est à même d'en ajuster les paramètres réactionnels sur la base de ses connaissances générales.

### Utilisation cosmétique

Le principe actif selon l'invention ou la composition selon l'invention est destiné à être utilisé sur des peaux saines, notamment des peaux saines déshydratées, en particulier comme repulpant, hydratant, et boosteur d'éclat.

Préférentiellement, l'utilisation cosmétique du principe actif selon l'invention ou de la composition selon l'invention vise un effet repulpant et/ou hydratant et/ou pour améliorer l'éclat du teint, plus préférentiellement pour augmenter le volume cellulaire cutané, et/ou l'expression de l'aquaporine 3 et/ou la synthèse du transporteur BGT-1 et/ou les facteurs naturels d'hydratation (FNH).

Les aquaporines, petits canaux intégrés aux membranes cellulaires, permettent la circulation dynamique et efficace de l'eau. L'aquaporine 3 est majoritairement présente dans la peau, tout particulièrement dans la membrane des kératinocytes. De la répartition optimale de ces canaux dans toutes les couches dépend le niveau d'hydratation de l'épiderme. En assurant le flux de trois milliards de molécules d'eau par seconde, elles garantissent un épiderme correctement irrigué jusqu'en surface.

Le transporteur à osmolytes BGT-1 (betaine-GABA transporter) est un régulateur de la teneur intracellulaire en bétaïne, un des osmolytes majoritaires de l'épiderme. En effet, un des mécanismes de défense des cellules contre le stress hydrique est l'ajustement osmotique. Les osmolytes sont de petites molécules solubles qui jouent un rôle dans la protection de la cellule contre le déficit hydrique. En condition de stress, les osmolytes s'accumulent au sein de la cellule grâce à l'activité de transporteurs spécifiques afin d'éviter la déperdition en eau et de maintenir les structures cellulaires. En plus de lutter contre la déshydratation des cellules, la bétaïne joue un rôle de chaperonne qui stabilise la structure et la fonction des protéines, notamment de celles impliquées dans les jonctions serrées.

Les facteurs naturels d'hydratation (FNH) regroupent un ensemble de substances hydrophiles intracornéocytaires contenues au niveau du *stratum corneum,* issus principalement de la dégradation de la filaggrine, et sont constitués essentiellement d'acides aminés (40%) dont le majoritaire est la sérine, de PCA (acide pyrrolidone-carboxylique) (12%), de lactate (12%), d'urée (7%) et d'ions inorganiques. Leur présence permet de capter l'eau libre, de la retenir au sein des cornéocytes et de jouer ainsi un rôle important dans le maintien des propriétés physiques du *stratum corneum.* Le lactate en particulier maintiendrait le pH acide de la couche cornée, un des paramètres importants garantissant l'intégrité et la cohésion du *stratum corneum.*

Le principe actif selon l'invention est particulièrement d'intérêt pour ses effets repulpant, hydratant et booster d'éclat cutané.

L'invention a donc également pour objet un procédé cosmétique de traitement de la peau pour un effet repulpant et/ou hydratant et/ou pour améliorer l'éclat du teint qui consiste en l'application topique sur la peau saine déshydratée d'une personne d'un tel principe actif selon l'invention ou d'une telle composition selon l'invention.

Le principe actif selon l'invention ou la composition selon l'invention permet ainsi d'améliorer l'effet repulpant et l'hydratation. En effet, chez des volontaires présentant une peau déshydratée au niveau du visage et un teint terne, après 42 jours d'applications biquotidiennes d'une composition cosmétique contenant le principe actif, le principe actif repulpe significativement la peau du visage. Cet effet s'accompagne d'un lissage du microrelief cutané et de l'amélioration des paramètres caractéristiques de l'éclat du teint des volontaires.

L'invention est à présent illustrée par des exemples non limitatifs de compositions selon l'invention et par des résultats d'efficacité.

### Exemples

### Exemple 1 : Principe actif selon l'invention (PA1)

Le principe actif de l'exemple 1 est obtenu à partir d'une lentille d'eau de l'espèce *Spirodela polyrhiza.* Le principe actif de l'exemple 1 est obtenu par le procédé suivant :
- Solubilisation d'au moins 50 g/L de *Spirodela polyrhiza* dans l'eau,
- Hydrolyse chimique de nature acide et hydrolyse enzymatique
- Séparation des phases solubles et insolubles et récupération de la phase soluble,
- Traitement thermique
- Purification et concentration de la phase soluble,
- Filtration et filtration stérilisante.

Le principe actif obtenu possède les caractéristiques suivantes :
- Teneur en Matières Sèches = 7,4 g/L
- Teneur en Sucres Neutres Totaux (selon la méthode de Dubois) = 2,4 g/L, soit 32%, en poids par rapport à la matière sèche
- Teneur en acides uroniques = 3,2g/l, soit 43%, en poids par rapport à la matière sèche
- Teneur en apiogalacturonanes = 55% en poids par rapport à la matière sèche
- Ratio acide galacturonique / apiose = 3,7
- Teneur en cendres minérales = 1,7 g/L (23% en poids par rapport à la matière sèche)
- pH = 3,5
- Liquide limpide, jaune très pâle, faible odeur

### Exemple 2 : Principe actif selon l'invention (PA2)

Le principe actif de l'exemple 2 est obtenu à partir d'une lentille d'eau de l'espèce *Spirodela polyrhiza.* Le principe actif de l'exemple 2 est obtenu par le procédé suivant :
- Solubilisation d'au moins 50g/L de *Spirodela polyrhiza* dans l'eau
- Hydrolyse chimique de nature acide,
- Séparation des phases solubles et insolubles et récupération de la phase soluble
- Purification et concentration de la phase soluble
- Filtration et filtration stérilisante.

Le principe actif obtenu possède les caractéristiques suivantes :
- Teneur en Matières Sèches = 6,6 g/L
- Teneur en Sucres Totaux (selon la méthode Dubois - gamme glucose) = 2,6g/L (39% en poids par rapport à la matière sèche)
- Teneur en acides uroniques = 2,2g/l (33% en poids par rapport à la matière sèche)
- Teneur en apiogalacturonanes = 40% en poids par rapport à la matière sèche
- Ratio acide galacturonique / apiose = 2,3
- pH = 3,5
- Liquide limpide, jaune clair faible odeur

### Exemple 3 : Principe actif hors invention (HI1)

Le principe actif de l'exemple 3 est obtenu à partir de fruit de *Pyrus malus,* comprenant des saccharides de type homogalacturonane, soit une chaîne d'acide galacturonique non ramifiée. Ce principe actif est obtenu selon le procédé suivant :
- Solubilisation de fruit de *Pyrus malus* dans l'eau,
- Hydrolyse enzymatique,
- Séparation des phases soluble et insoluble,
- Traitement thermique,
- Purification et concentration de la phase soluble
- Filtration et filtration stérilisante.

Le principe actif selon l'exemple 3 présente les caractéristiques suivantes :
- Teneur en matière sèche de 164 g/L
- Teneur en acides uroniques = 126g/l, soit 77% en poids par rapport à la matière sèche
- Identification des sucres présents : acide galacturonique
- Teneur en apioses liés : 0g/l
- Teneur en apiogalacturonanes = 0g/l
- pH = 3,1
- Liquide limpide de couleur jaune orangé

Malgré une structure polysaccharidique avec une chaîne linéaire d'acides galacturoniques, ce principe actif diffère du principe actif selon l'invention en ce qu'il ne contient pas d'apioses et pas d'apiogalacturonanes.

### Exemple 4 : Principe actif hors invention (HI2)

Le principe actif de l'exemple 4 est obtenu à partir de racines *d'Ophiopogon japonicus* et comprend des saccharides de type fructosanes, soit une chaîne de fructose. Ce produit est obtenu selon le procédé suivant :
- Solubilisation de poudre de racines *d'Ophiopogon japonicus* dans l'eau
- Hydrolyse enzymatique
- Séparation des phases soluble et insoluble
- Traitement thermique
- Purification
- Filtration et filtration stérilisante

Le produit obtenu selon l'exemple 4 présente les caractéristiques suivantes :
- Teneur en matière sèche de 123 g/L
- Teneur en sucres totaux (selon la méthode de Dubois avec gamme de fructose) = 115 g/L, soit 93% en poids par rapport à la matière sèche.
- Identification des sucres présents : fructose et glucose
- Teneur en apioses = 0g/l
- Teneur en apiogalacturonanes = 0g/l
- pH = 5,1.
- Liquide limpide de couleur jaune

Cet exemple diffère du principe actif selon l'invention en ce qu'il ne contient pas d'apiogalacturonanes.

### Exemple 5 : Composition selon l'invention

Un exemple de formulation d'un sérum comprenant le principe actif selon l'invention sous forme de gel est présenté dans le tableau 1 suivant.

**[Tableau 1]**

| | **Ingrédients** | % |
|---|---|---|
| A | Eau purifiée | qsp 100 |
| B | Polyquaternium-37 & Eau | 0,70 |
| C | Butylene Glycol | 5,00 |
| | Glyceryl Caprylate | 1,00 |
| | 1,2-Hexanediol | 1,00 |
| | Glycerin | 1,00 |
| | Glycereth-26 | 4,00 |
| | Isopentyldiol | 4,00 |
| | Caprylic/Capric/Succinic Triglyceride | 1,50 |
| D | **PRINCIPE ACTIF INVENTION** | 3,00 |
| E | Soude 7% | qs pH |

La composition de l'exemple 5 peut notamment être obtenue par le procédé suivant :
- Saupoudrer B dans A, agiter jusqu'à obtention d'un gel homogène.
- Ajouter une à une les matières de la phase C sous agitation modérée.
- Ajouter D sous agitation modérée.
- Ajuster le pH à 6,0 - 6,5 avec E.

La composition est alors sous forme d'un gel opalescent souple, blanc et brillant présentant un pH à 1 mois égal à 6,2, une viscosité (C/5rpm) égale à 16 600 cP.

### Exemple 6 : Composition selon l'invention

Un exemple de formulation comprenant le principe actif selon l'invention sous forme d'une crème est présenté dans le tableau 2 suivant.

**[Tableau 2]**

| | **Ingrédients** | % |
|---|---|---|
| A1 | Eau Purifiée | qsp 100 |
| | Butylene Glycol | 3,00 |
| A2 | Glycerin | 2,00 |
| | Xanthan Gum | 0,15 |
| B | Cetearyl Alcohol & Ceteareth-33 | 4,00 |
| | Myristyl Alcohol & Myristyl Glucoside | 1,50 |
| | C10-18 Triglycerides | 2,00 |
| | Caprylic/Capric Triglyceride | 5,00 |
| | Dicaprylyl Carbonate | 5,00 |
| | Isopropyl Myristate | 4,00 |
| | Ricinus Communis (Castor) Seed Oil | 3,00 |
| | Dimethicone | 1,00 |
| | Tocopherol & Helianthus Annuus (Sunflower) Seed Oil | 0,05 |
| C | Sodium Polyacrylate | 1,00 |
| D | **PRINCIPE ACTIF INVENTION** | 3,00 |

La composition de l'exemple 6 peut notamment être obtenue par le procédé suivant :
- Ajouter A2 dans A1 sous agitation modérée, agiter jusqu'à obtention d'un gel homogène et chauffer à 80°C
- Placer B sous agitation et chauffer à 80°C
- Emulsionner B dans A sous agitation cisaillante pendant 10 minutes
- A 40°C, sous agitation modérée, ajouter C puis D.

La composition est alors sous forme d'une émulsion épaisse, blanche et brillante présentant un pH à 1 mois égal à 6, une viscosité (C/5rpm) égal à 318 000 cP.

### Exemple 7 : Composition selon l'invention

Un exemple de formulation comprenant le principe actif selon l'invention sous forme d'une crème est présenté dans le tableau 3 suivant.

**[Tableau 3]**

| | **Ingrédients** | % |
|---|---|---|
| A1 | Eau Purifiée | qsp 100% |
| | Butylene Glycol | 2,00 |
| | Solution Soude 28% | 0,55 |
| A2 | Isopentyldiol | 2,00 |
| | Glycerin | 2,00 |
| | Xanthan Gum | 0,15 |
| B | C20-22 Alkyl Phosphate & C20-22 Alcohols | 3,00 |
| | Cetearyl Alcohol | 1,00 |
| | C10-18 Triglycerides | 2,00 |
| | Squalane | 4,00 |
| | Isononyl Isononanoate | 5,00 |
| | Caprylic/Capric Triglyceride | 7,50 |
| | Isocetyl Stéarate | 3,25 |
| | Caprylyl Methicone | 4,00 |
| C | Hydroxyethyl Acrylate/Sodium Acryloyldimethyl Taurate Copolymer & Polyisobutene & PEG-7 Trimethylolpropane Coconut Ether | 0,75 |
| D | **PRINCIPE ACTIF INVENTION** | 3,00 |

La composition de l'exemple 7 peut notamment être obtenue par le procédé suivant :
- Ajouter A2 dans A1 sous agitation modérée, agiter jusqu'à obtention d'un gel homogène et chauffer à 80°C
- Placer B sous agitation et chauffer à 80°C
- Emulsionner B dans A sous agitation cisaillante pendant 10 minutes.
- A 40°C, sous agitation modérée, ajouter C puis D.

La composition est alors sous forme d'une émulsion souple, blanche et brillante présentant un pH à 1 mois égal à 4,8, une viscosité (C/5rpm) égal à 58 000 cP.

### Exemple 8 : Composition selon l'invention

Un exemple de formulation comprenant le principe actif selon l'invention sous forme d'un fond de teint est présenté dans le tableau 4 suivant.

**[Tableau 4]**

| | **Ingrédients** | % |
|---|---|---|
| A1 | Eau Purifiée | qsp 100% |
| | Sodium Gluconate | 0,20 |
| | Glycerin | 2,00 |
| | Butylene Glycol | 3,00 |
| A2 | Ci 77499 (Iron Oxides) & Silica | 0,10 |
| | Ci 77491 (Iron Oxides) & Silica | 0,50 |
| | Ci 77891 (Titanium Dioxide) & Silica | 6,50 |
| | Ci 77492 (Iron Oxides) & Silica | 1,00 |
| B | Disodium Cetearyl Sulfosuccinate | 1,00 |
| | Glyceryl Stéarate | 1,50 |
| | Cetearyl Alcohol | 0,50 |
| | Pentaerythrityl Distearate | 1,20 |
| | Pentaerythrityl Tetrabehenate | 0,80 |
| | Dipentaerythrityl Hexacaprylate/Hexacaprate | 3,00 |
| | Cocoglycerides | 3,00 |
| | Isocetyl Stéarate | 3,00 |
| | Propanediol Dicaprylate | 5,00 |
| | Diisopropyl Adipate | 4,00 |
| | Dimethicone | 4,00 |
| | VP/Hexadecene Copolymer | 2,00 |
| | Polyacrylate Crosspolymer-6 | 0,30 |
| | Hydroxyethyl Acrylate/Sodium Acryloyldimethyl Taurate Copolymer | 0,30 |
| C | **PRINCIPE ACTIF INVENTION** | 3,00 |

La composition de l'exemple 8 peut notamment être obtenue par le procédé suivant :
- Disperser A2 dans A1 sous agitation cisaillante jusqu'à homogénéité et chauffer à 80°C
- Placer B sous agitation et chauffer à 80°C
- Emulsionner B dans A sous agitation cisaillante pendant 10 minutes.
- A 40°C, sous agitation modérée, ajouter C.

La composition est alors sous forme d'une émulsion épaisse, de couleur beige rosé présentant un pH à 1 mois égal à 5,9, une viscosité (C/5rpm) égal à 123 000 cP.

### Mécanisme d'action de l'efficacité du principe actif selon l'invention

Essai 1 - Effet du principe actif selon l'invention sur l'expression des aquaporines 3.

L'expression de l'aquaporine 3 (AQP3) a été évaluée par PCR quantitative sur des kératinocytes humains normaux soumis à un stress hydrique.

A J0, les kératinocytes humains sont ensemencés et incubés à 37°C dans une atmosphère contenant 5% de CO2. A J4, les cellules sont traitées avec le principe actif selon l'invention à 0,5% (v/v) puis incubées à 37°C sous 5% de CO2 dans un environnement normal ou dans un environnement sec pendant 24h. A J5, les cellules sont récupérées et les ARN totaux extraits. Les ARNs ont été reverse-transcrits et les ADNs complémentaires obtenus ont été analysés par la technique de PCR quantitative.

Les résultats sont présentés dans le tableau 5 ci-après.

**[Tableau 5]**

| | **Expression de AQP3 (%)** | **AQP3 / témoin déshydratés (%)** |
|---|---|---|
| **Kératinocytes normaux** | | |
| Témoin | 100 | |
| PRINCIPE ACTIF SELON L'INVENTION (PA1) 0,5% | 112 | |

| **Kératinocytes déshydratés** | | |
|---|---|---|
| Témoin | 80 | |
| PRINCIPE ACTIF SELON L'INVENTION (PA1) 0,5% | 113^{∗} | **+41** |
| PRINCIPE ACTIF SELON L'INVENTION (PA2) 0,5% | 110^{∗} | **+38** |
| EXTRAIT HORS INVENTION (HI1) 0,025% | 78 | **0** |

Le modèle déshydraté présente une expression de l'aquaporine 3 significativement réduite de 20%. Testé à 0,5% sur des kératinocytes déshydratés, le principe actif selon l'invention augmente significativement l'expression d'aquaporine 3 de 41%. Le principe actif selon l'invention stimule ainsi la synthèse d'un des médiateurs clés de l'hydratation.

Essai 2 - Effet du principe actif selon l'invention sur l'expression des BGT-1.

La synthèse de BGT-1 a été évaluée par imunohistofluorescence sur des explants de peau humaine normaux soumis à un stress hydrique.

Pendant plusieurs jours, les explants sont incubés à 37°C sous 5% de CO2 dans un environnement normal ou dans un environnement sec (stress hydrique). Les explants sont traités en topique avec le principe actif selon l'invention formulé à 0,5% et 1,0% (V/V) ou avec le placebo.

A la fin des traitements, les explants sont récupérés et congelés. Des coupes (4 µm) sont ensuite réalisées à l'aide d'un cryostat, afin d'analyser la synthèse de BGT-1 par immunohistofluorescence à l'aide d'anticorps anti-BGT-1.

La synthèse de BGT-1 est proportionnelle à l'intensité de la fluorescence. L'analyse statistique est réalisée avec le test non paramétrique de Mann-Whitney.

Les résultats sont présentés dans le tableau 6 ci-après.

**[Tableau 6]**

| | **Synthèse de BGT-1 (UA)** | **Capacité à restaurer la synthèse de BGT-1 (%)** |
|---|---|---|
| **Explants normaux** | | |
| Témoin | 24 | |
| PRINCIPE ACTIF SELON L'INVENTION (PA1) 1,0% | 24 | |

| **Explants déshydratés** | | |
|---|---|---|
| Témoin | 19 | |
| Placebo | 20^{ns} | |
| PRINCIPE ACTIF SELON L'INVENTION (PA1) 0,5% | 22 ^{s} | **+60** |
| PRINCIPE ACTIF SELON L'INVENTION (PA1) 1,0% | 24 ^{s} | **+100** |

Le modèle d'explant déshydraté est caractérisé par une diminution significative de BGT-1 de 21%. Testé à 1% sur modèle déshydraté, le principe actif selon l'invention restaure significativement la synthèse de BGT-1. Le principe actif selon l'invention permet ainsi de rétablir l'équilibre osmotique cutané.

Essai 3 - Effet du principe actif selon l'invention sur le volume cellulaire.

Le suivi du volume des kératinocytes humains normaux a été réalisé avant et pendant l'application d'un stress hyper-osmotique pour mimer un état de déshydratation.

A J0, les kératinocytes sont ensemencés dans du milieu de culture et incubés à 37°C dans une atmosphère contenant 5% de CO2. Ils sont ensuite traités avec le principe actif selon l'invention à 0,5% (V/V) et incubés à 37°C dans une atmosphère contenant 5% de CO2. Après plusieurs jours, les kératinocytes sont marqués avec une sonde fluorescente, la calcéine, puis replacés dans du milieu de culture contenant ou non le principe actif selon l'invention à 0,5% (V/V).

La visualisation est ensuite réalisée à l'aide d'un microscope couplé à une caméra et à un système d'analyse d'images. Des piles d'images des cellules vivantes sont prises afin de pouvoir visualiser les cellules en 3-dimensions, toutes les deux minutes sur une durée de 18 minutes au total, divisée en 2 phases. Une 1ère phase (0 à 6 minutes) en condition iso-osmotique (milieu de culture normal), et une 2ème phase (8 à 18 minutes) en stress hyper-osmotique.

Une analyse quantitative du volume cellulaire a été réalisée grâce à un script d'analyse d'images développé par l'inventeur. Les résultats sont exprimés en pourcentage du volume initial des cellules.

Les résultats sont présentés dans le tableau 7 ci-après.

**[Tableau 7]**

| | | **Volume cellulaire (%)** | | **Volume cellulaire / témoin (%)** |
|---|---|---|---|---|
| Temps (min) | Condition osmotique | Témoin | PRINCIPE ACTIF SELON L'INVENTIO N (PA1) 0,5% | PRINCIPE ACTIF SELON L'INVENTION/Témoin |
| 0 | Phase 1 : condition iso-osmotique | 100 | 100 | |
| 2 | | 94 | 95 | |
| 4 | | 93 | 96 | |
| 6 | | 93 | 95 | |
| 8 | Phase 2 : stress hyper-osmotique | 58 | 66*** | **+ 14** |
| 10 | | 58 | 66*** | **+ 14** |
| 12 | | 58 | 66*** | **+ 14** |
| 14 | | 57 | 66*** | **+ 16** |
| 16 | | 57 | 66*** | **+ 16** |
| 18 | | 57 | 65*** | **+ 14** |

Après un stress hyper-osmotique, le volume cellulaire moyen est réduit de 43%. Lorsque des kératinocytes sont traités au préalable avec le principe actif selon l'invention à 0,5%, le volume cellulaire après un choc hyper-osmotique n'est réduit que de 34%. Ainsi, le principe actif selon l'invention limite significativement de 16% la perte de volume cellulaire à la suite d'un choc hyper-osmotique. Le principe actif selon l'invention permet donc de retenir l'eau intracellulaire.

Essai 4 - Effet du principe actif selon l'invention à capter l'eau dans l'épiderme.

L'essai 4 vise à démontrer la capacité du principe actif selon l'invention à capter l'eau au coeur de l'épiderme, et de comparer cette efficacité à deux exemples hors invention. Pour cela, l'analyse cible la pénétration et le maintien de l'eau deutérée (D2O) apportée de façon exogène dans la peau 30 minutes et 3 heures après application. Les mesures sont réalisées par microspectroscopie Raman à différentes profondeurs pendant 10 minutes.

L'apport en eau exogène a été effectué par application à la surface de la peau d'un patch contenant de l'eau deutérée (D2O) ; le marquage permettant de suivre spécifiquement sa pénétration dans la peau. Cette étude a été réalisée sur 7 volontaires sains caucasiens de sexe féminin, âgés de 45 à 66 ans et présentant une peau déshydratée au niveau des avant-bras.

A T0, les 4 émulsions (émulsion placebo, émulsion contenant 3% du principe actif de l'invention dans l'exemple 1, émulsion contenant 0.12% de l'extrait hors invention selon l'exemple 3, émulsion contenant 0.12% de l'extrait hors invention selon l'exemple 4) sont appliquées sur les volontaires par massage jusqu'à pénétration complète. A T3 heures, un patch occlusif contenant du D2O est appliqué pendant 30 minutes, sur les zones préalablement traitées avec les émulsions. Après 30 minutes, le patch est retiré et l'eau résiduelle est éliminée. Les mesures par microspectroscopie Raman sont réalisées.

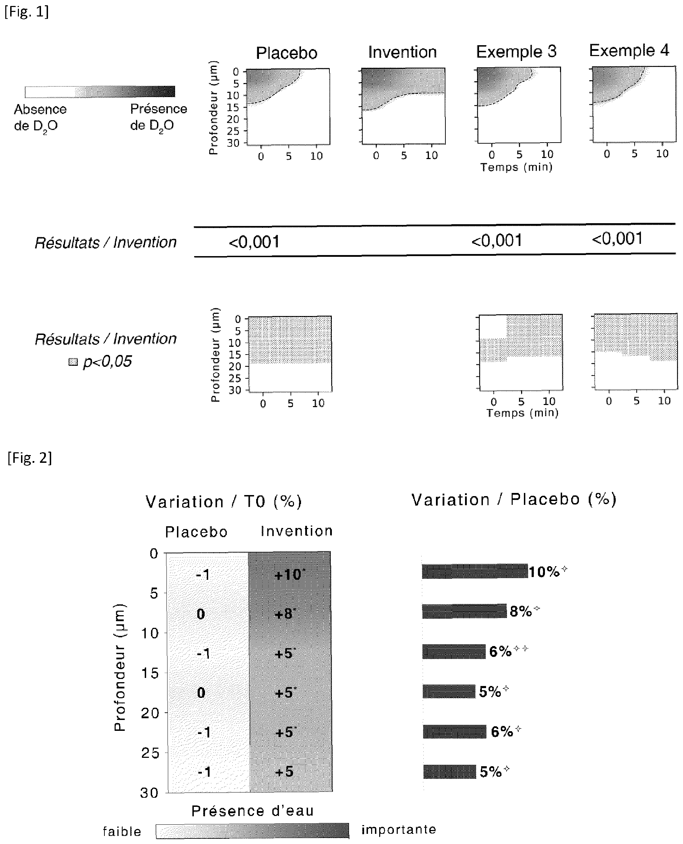

Les résultats sur la quantité de D2O dans l'épiderme pour les 4 émulsions sont présentés en Figure 1.

En comparaison au placebo et aux extraits hors invention des exemples 3 et 4, le principe actif selon l'invention présente un effet significativement différent. L'eau pénètre en quantité plus importante, plus profondément et reste plus longtemps dans la peau. Ces résultats traduisent ainsi *in vivo* le pouvoir hygroscopique supérieur du principe actif selon l'invention par rapport aux extraits hors invention.

### Evaluation in vivo de l'activité biologique du principe actif selon l'invention

Essai 5 - Etude du contenu en eau totale dans l'épiderme.

L'étude vise à évaluer *in vivo* l'effet immédiat et l'effet long terme du principe actif selon l'invention formulé à 3% en émulsion sur le contenu en eau totale de l'épiderme en comparaison à une formule placebo.

L'effet immédiat du principe actif selon l'invention a été étudié sur 19 volontaires sains caucasiens, de sexe féminin, âgés de 35 à 65 ans, présentant une peau déshydratée au niveau du visage, un microrelief irrégulier au niveau de la patte d'oie ainsi qu'un teint terne.

L'effet long terme du principe actif selon l'invention a été étudié sur 17 volontaires sains caucasiens de sexe féminin, âgés de 44 à 65 ans présentant une peau déshydratée au niveau du visage, un microrelief irrégulier au niveau de la patte d'oie ainsi qu'un teint terne.

Les mesures sont réalisées par microspectroscopie Raman sur des zones symétriques au niveau des joues, avant puis 3h après une unique application des produits à l'étude en hémi-visage (effet immédiat), et avant et après 21 et 42 jours de traitement biquotidien en hémi-visage (effet long terme). Le contenu en eau totale a été évaluée en étudiant le rapport des bandes Raman vOH-total/vCH.

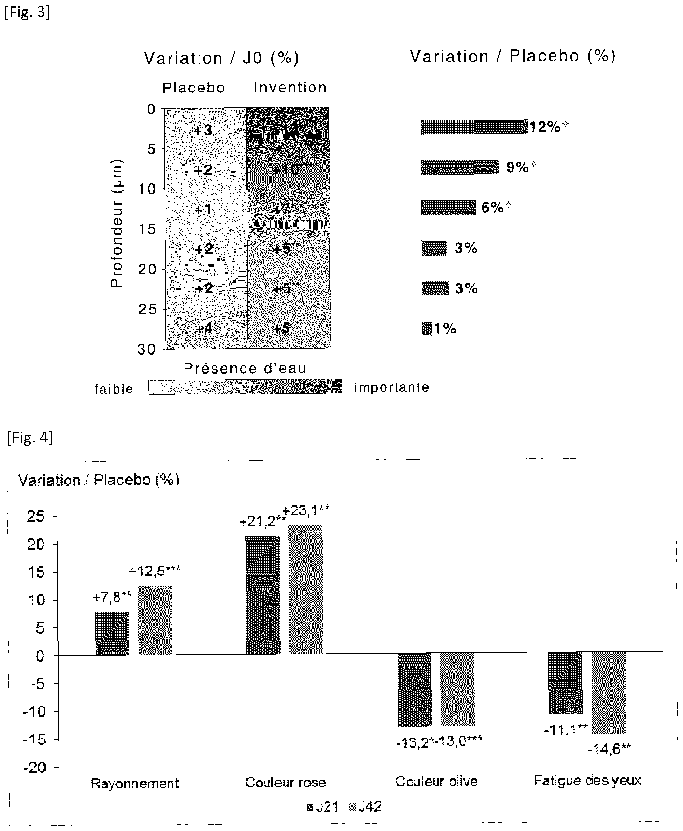

Les résultats correspondant à l'effet du principe actif selon l'invention, formulé à 3% en émulsion, sur la quantité d'eau totale au niveau de l'épiderme de volontaires caucasiens sont présentés sur la figure 2 pour l'effet immédiat et la figure 3 pour l'effet long terme.

Dès 3 heures après une application unique, le principe actif selon l'invention formulé à 3% en émulsion, augmente significativement la quantité d'eau totale retenue dans l'épiderme. Ces augmentations ont été observées chez 60% des volontaires. Cet effet s'intensifie après 42 jours d'application biquotidienne. En effet, le principe actif selon l'invention entraîne une augmentation significative de la quantité d'eau totale au niveau du *stratum corneum.* Ces augmentations ont été observées chez plus de 75% des volontaires.

Essai 6 - Etude des facteurs naturels d'hydratation.

L'objectif de cette étude est d'évaluer *in vivo,* en comparaison à une formule placebo, l'effet du principe actif selon l'invention formulé à 3% en émulsion sur les facteurs naturels d'hydratation, un ensemble de substances hydrophiles intracornéocytaires (e.g lactate, urée, ion inorganique, acide aminé) présentes dans le *stratum corneum.*

L'étude a été réalisée sur deux groupes de volontaires sains caucasiens, de sexe féminin, présentant une peau déshydratée au niveau du visage, un microrelief irrégulier au niveau de la patte d'oie ainsi qu'un teint terne. Un groupe placebo constitué de 18 sujets âgés de 35 à 67 ans et un groupe le principe actif selon l'invention constitué de 20 sujets âgés de 36 à 67 ans. Les volontaires ont appliqué le principe actif selon l'invention ou le placebo, sur l'ensemble du visage, de façon biquotidienne, pendant 42 jours. Le lactate a été dosé par fluorimétrie à partir de prélèvements réalisés au niveau des joues.

Les résultats correspondant à l'effet du principe actif selon l'invention formulé à 3% en émulsion sur la concentration en lactate, sont présentés dans le tableau 8.

**[Tableau 8]**

| | Variation J21/J0 | Variation J42/J0 |
|---|---|---|
| PLACEBO | -1,0^{ns} | -0,9^{ns} |
| PRINCIPE ACTIF SELON L'INVENTION (PA1) 3% | +31,7^{s} | +50,2^{s} |
| Comparaison PRINCIPE ACTIF SELON L'INVENTION / Placebo | +32,7% | +51,2% |

Testé à 3% sur panel caucasien, le principe actif selon l'invention augmente significativement la concentration en lactate en comparaison au placebo. En effet, après 21 jours de traitement biquotidien, la concentration en lactate est significativement augmentée de 32,7%. Cet effet s'intensifie après 42 jours de traitement avec une augmentation significative de 51,2%.

### Essai 7 - Etude des pertes hydriques

L'objectif de cette étude est d'évaluer *in vivo* l'effet du principe actif selon l'invention formulé à 3% en émulsion sur les pertes hydriques après une application unique, en comparaison avec une formule placebo. L'étude a été réalisée sur 16 volontaires sains caucasiens, de sexe féminin, âgés de 35 à 65 ans, présentant une peau déshydratée au niveau du visage, un microrelief irrégulier au niveau de la patte d'oie ainsi qu'un teint terne. Les mesures ont été réalisées à l'aide d'un Tewamètre^{®} (Courage & Khazaka) sur des zones symétriques au niveau des joues, avant puis 3 et 6 heures après une application unique des produits en hémi-visage.

Les résultats correspondant à la capacité du principe actif selon l'invention, formulé à 3% en émulsion, à limiter les pertes en eau sont présentés dans le tableau 9.

**[Tableau 9]**

| | Variation T3H/T0 | Variation T6H/T0 |
|---|---|---|
| PLACEBO | +0,4^{ns} | +2,6^{ns} |
| PRINCIPE ACTIF SELON L'INVENTION (PA1) 3% | -11,5^{s} | -13,1^{s} |
| Comparaison PRINCIPE ACTIF SELON L'INVENTION / Placebo | -11,9% | -15,7% |

Dès 3 heures après une application unique, le principe actif selon l'invention formulé à 3%, permet de réduire les pertes hydriques de 11,9% en comparaison au placebo. Cette action s'amplifie 6 heures après l'application avec une réduction des pertes hydriques de 15,7%, effet observé chez 69% des sujets. Le principe actif selon l'invention permet donc de retenir l'eau au coeur de l'épiderme.

### Evaluation de l'efficacité cosmétique du principe actif selon l'invention

### Essai 8 - Effet hydratant immédiat du principe actif selon l'invention

L'objectif de cette étude est d'évaluer *in vivo* l'effet immédiat du principe actif selon l'invention formulé à 3% en émulsion, sur l'hydratation des couches profondes et superficielles de la peau de volontaires caucasiens en comparaison à une formule placebo. Cette étude a été réalisée sur 16 volontaires sains caucasiens, de sexe féminin, d'âge compris entre 35 et 65 ans, présentant une peau déshydratée au niveau du visage, un microrelief irrégulier au niveau de la patte d'oie et un teint terne. L'effet hydratant immédiat au niveau de l'épiderme et du derme supérieur a été évalué par mesure du taux d'hydratation à l'aide d'un MoistureMeter-D (Delfin Technologies). La capacité du principe actif selon l'invention à hydrater de manière immédiate le *stratum corneum* a quant à elle été évaluée via la mesure du taux d'hydratation à l'aide d'un Cornéomètre^{®} CM825 (Courage & Khazaka). Les mesures ont été réalisées 3 et 6 heures après une application unique du principe actif selon l'invention et du placebo en hémi-visage.

Les résultats présentant l'effet immédiat du principe actif selon l'invention formulé à 3% en émulsion, sur l'hydratation de l'épiderme et du derme supérieur est présenté dans le tableau 10 et sur l'hydratation du *stratum corneum* dans le tableau 11.

**[Tableau 10]**

| Sur l'épiderme et le derme supérieur | Variation T3H/T0 | Variation T6H/T0 |
|---|---|---|
| PLACEBO | -1,5^{ns} | -1,6^{ns} |
| PRINCIPE ACTIF SELON L'INVENTION (PA1) 3% | +10,4^{s} | +14,2^{s} |
| Comparaison PRINCIPE ACTIF SELON L'INVENTION / Placebo | +11,9% | +15,8% |

Dès 3 heures après une application unique, en comparaison au placebo, le principe actif selon l'invention formulé à 3% en émulsion augmente significativement l'hydratation de la peau au niveau de l'épiderme et du derme supérieur de 11,9%. Cet effet se prolonge 6 heures après l'application avec une augmentation de l'hydratation égale à 15,8%. Ces résultats ont été observés chez respectivement 63% et 81% des sujets.

**[Tableau 11]**

| Sur le Stratum cornéum | Variation T3H/T0 | Variation T6H/T0 |
|---|---|---|
| PLACEBO | +0,4^{ns} | +4,7^{ns} |
| PRINCIPE ACTIF SELON L'INVENTION (PA1) 3% | +18,2^{s} | -29,8^{s} |
| Comparaison PRINCIPE ACTIF SELON L'INVENTION / Placebo | +17,8% | +25,1% |

le principe actif selon l'invention améliore immédiatement l'hydratation de surface chez des volontaires. En effet, dès 3 heures après une application unique, le principe actif selon l'invention formulé à 3% en émulsion augmente l'hydratation du stratum cornéum de 17,8%. Cette action se prolonge et s'intensifie 6 heures après le traitement avec une amélioration significative de l'hydratation de surface de 25,1%. Cet effet est observé chez 88% des volontaires.

Ainsi, le principe actif selon l'invention formulé à 3% en émulsion, améliore l'hydratation immédiate du derme supérieur jusqu'au *stratum corneum* dès 3 heures après une application unique chez des volontaires caucasiens.

### Essai 9 - Effet hydratant à long terme du principe actif selon l'invention

L'objectif de cette étude est d'évaluer l'effet long terme du principe actif selon l'invention sur l'hydratation des couches profondes et superficielles de l'épiderme de volontaires caucasiens et asiatiques, en comparaison à une formule placebo.

Cette étude a été réalisée sur un panel caucasien de 17 volontaires sains caucasiens, de sexe féminin, d'âge compris entre 44 et 65 ans et sur un panel asiatique de 63 volontaires sains, de sexe féminin, d'âge compris entre 41 et 65 ans, présentant une peau déshydratée au niveau du visage, un microrelief irrégulier au niveau de la patte d'oie et un teint terne.

L'hydratation en profondeur a été évaluée par mesure du taux d'hydratation à l'aide d'un MoistureMeter-D et l'hydratation de surface via la mesure du taux d'hydratation à l'aide d'un Cornéomètre^{®} CM825.

Les volontaires caucasiens ont appliqué le principe actif selon l'invention formulé à 3% en émulsion et le placebo en hémi-visage de manière biquotidienne pendant 21 et 42 jours.

Les volontaires asiatiques ont été répartis en 2 groupes ayant respectivement appliqué en visage entier la formule contenant le principe actif selon l'invention à 3% ou la formule placebo de manière biquotidienne pendant 21 et 42 jours. Le groupe recevant le principe actif selon l'invention est constitué de 32 volontaires et le groupe recevant le placebo de 31 volontaires.

Les résultats présentant l'effet à long terme sur l'hydratation de l'épiderme et du derme supérieur et sur l'hydratation de surface de volontaires caucasiens sont respectivement présentés dans les tableaux 12 et 13. Les résultats présentant l'effet à long terme sur l'hydratation de surface de volontaires asiatiques est présenté dans le tableau 14.

**[Tableau 12]**

| Hydratation de l'épiderme et du derme supérieur | Variation J21 / J0 | Variation J42 / J0 |
|---|---|---|
| Panel caucasien | | |
| PLACEBO | +10,4^{s} | +2,4^{ns} |
| PRINCIPE ACTIF SELON L'INVENTION (PA1) 3% | +21,0^{s} | +18,0^{s} |
| Comparaison PRINCIPE ACTIF SELON L'INVENTION / Placebo | +10,6% | +15,5% |

Après 21 jours de traitement, le principe actif selon l'invention augmente significativement l'hydratation de l'épiderme et du derme supérieur de 10,6%. Cet effet se prolonge et s'intensifie après 42 jours de traitement avec une amélioration significative de 15,5%. Cette augmentation est observée chez 88% des volontaires.

**[Tableau 13]**

| Hydratation du stratum cornéum Panel caucasien | Variation J21 / J0 | Variation J42 / J0 |
|---|---|---|
| PLACEBO | +4,5^{ns} | +2,8^{ns} |
| PRINCIPE ACTIF SELON L'INVENTION (PA1) 3% | +10,5^{s} | +12,8^{s} |
| Comparaison PRINCIPE ACTIF SELON L'INVENTION / Placebo | +5,9% | +10,0% |

Après 21 jours de traitement, le principe actif selon l'invention augmente significativement l'hydratation du stratum cornéum des volontaires caucasiens de 5,9%. Cet effet se prolonge et s'intensifie après 42 jours de traitement avec une amélioration significative de 10,0%. Cette augmentation est observée chez 76% des volontaires.

**[Tableau 14]**

| Hydratation du stratum cornéum Panel asiatique | Variation J21 / J0 | Variation J42 / J0 |
|---|---|---|
| PLACEBO | -0,5^{s} | -1,9^{ns} |
| PRINCIPE ACTIF SELON L'INVENTION (PA1) 3% | +12,4^{s} | +20,6^{s} |
| Comparaison PRINCIPE ACTIF SELON L'INVENTION / Placebo | +12,9% | +22,5% |

Après 21 jours de traitement, le principe actif selon l'invention augmente significativement l'hydratation du stratum cornéum des volontaires asiatiques de 12,9%. Cet effet se prolonge et s'intensifie après 42 jours de traitement avec une amélioration significative de 22,5%. Cette augmentation est observée chez 97% des volontaires.

Ainsi, le principe actif selon l'invention formulé à 3% en émulsion, améliore l'hydratation des couches superficielles de l'épiderme jusqu'au derme supérieur après 6 semaines de traitement biquotidien chez des volontaires caucasiens et asiatiques.

### Essai 10 - Effet repulpant du principe actif selon l'invention

L'objectif de cette étude est d'évaluer *in vivo* l'effet repulpant du principe actif selon l'invention formulé à 3% en émulsion, en comparaison à une formule placebo sur des volontaires caucasiens. Le panel est composé de deux groupes de volontaires sains présentant une peau déshydratée au niveau du visage, un microrelief irrégulier au niveau de la patte d'oie ainsi qu'un teint terne. Un 1^{er} groupe traité par le principe actif selon l'invention comprend 20 volontaires âgés de 36 à 67 ans et un 2^{ème} groupe traité par le placebo comprend 18 volontaires âgés de 35 à 67 ans. L'effet repulpant a été étudié par mesure de la volumétrie du visage (système EvaFACE^{3D}-S5, Eotech) après 21 et 42 jours d'application biquotidienne du principe actif selon l'invention ou du placebo sur l'ensemble du visage.

Les résultats correspondant à l'effet du principe actif selon l'invention formulé à 3% en émulsion, sur le paramètre volume positif est présenté dans le tableau 15.

**[Tableau 15]**

| | Variation J21 / J0 | Variation J42 / J0 |
|---|---|---|
| PLACEBO | -0,8^{s} | -3,6^{ns} |
| PRINCIPE ACTIF SELON L'INVENTION (PA1) 3% | +31,2^{s} | +43,7^{s} |
| Comparaison PRINCIPE ACTIF SELON L'INVENTION / Placebo | +32,0% | +47,3% |

Ainsi, le principe actif selon l'invention, en comparaison au placebo, repulpe significativement la peau du visage de volontaires caucasiens. En effet, après 21 jours d'application biquotidienne sur l'ensemble du visage, le principe actif selon l'invention formulé à 3% en émulsion, améliore significativement de 32,0% le paramètre volume positif, caractéristique d'un effet repulpant. Cet effet a été observé chez 82% des volontaires. Cet effet se prolonge et s'intensifie après 42 jours de traitement où l'augmentation du paramètre volume positif est égale à 47,3%. Cette augmentation est visible chez 67% des volontaires.

### Essai 11 - Effet lissant du principe actif selon l'invention

L'objectif de cette étude est d'évaluer *in vivo* l'effet lissant du principe actif selon l'invention formulé à 3% en émulsion, en comparaison à une formule placebo sur des volontaires asiatiques. Le panel était composé de deux groupes de volontaires sains présentant une peau déshydratée au niveau du visage, un microrelief irrégulier au niveau de la patte d'oie et sous les yeux ainsi qu'un teint terne. Un 1^{er} groupe recevant le principe actif selon l'invention comprend 32 volontaires âgés de 43 à 64 ans et un 2^{ème} groupe recevant le placebo de 31 volontaires âgés de 41 à 65 ans.

L'effet lissant a été évalué par scorage clinique par un dermatologue avant et après application du principe actif selon l'invention ou du placebo sur l'ensemble du visage, de façon biquotidienne pendant 42 jours.

Les résultats correspondant à l'effet lissant, évalué par scorage clinique au niveau du dessous de l'œil, du principe actif selon l'invention formulé à 3% en émulsion, est présenté dans le tableau 16.

**[Tableau 16]**

| | Variation J21 / J0 | Variation J42 / J0 |
|---|---|---|
| PLACEBO | -1,6^{s} | -4,0^{ns} |
| PRINCIPE ACTIF SELON L'INVENTION (PA1) 3% | -5,6^{s} | -11,4^{s} |
| Comparaison PRINCIPE ACTIF SELON L'INVENTION / Placebo | -4,0% | -7,4% |

Le principe actif selon l'invention, en comparaison au placebo, présente un effet lissant significatif sur la zone du contour de l'œil chez des volontaires asiatiques. En effet, après 21 jours d'application biquotidienne sur l'ensemble du visage, le principe actif selon l'invention formulé à 3% en émulsion, diminue significativement de 4,0% le stade de rides sous les yeux. Ces effets se prolongent après 42 jours de traitement avec une diminution des ridules sous les yeux de 7,4%. Cet effet est observé chez 66% des volontaires.

Essai 12 - Effet du principe actif selon l'invention sur l'éclat du teint.

L'objectif de cette étude est d'évaluer *in vivo* l'effet sur l'éclat du teint du principe actif selon l'invention formulé à 3% en émulsion, en comparaison à une formule placebo sur des volontaires caucasiens et asiatiques, après 21 et 42 jours d'application biquotidienne du principe actif selon l'invention ou du placebo sur l'ensemble du visage.

Les panels caucasien et asiatique étaient composés de deux groupes de volontaires sains présentant une peau déshydratée au niveau du visage, un microrelief irrégulier au niveau de la patte d'oie et sous les yeux ainsi qu'un teint terne.

Dans le panel caucasien, un 1^{er} groupe recevant le principe actif selon l'invention est composé de 20 volontaires âgés de 36 à 67 ans et un 2^{ème} groupe recevant le placebo de 18 volontaires âgés de 35 à 67 ans.

Dans le panel asiatique, un 1^{er} groupe recevant le principe actif selon l'invention de 32 volontaires âgés de 43 à 64 ans et un 2^{ème} groupe recevant le placebo de 31 volontaires âgés de 41 à 65 ans.

L'éclat du teint a été évalué par scorage clinique par deux experts entraînés (panel caucasien) ou un dermatologue (panel asiatique), avant et après 21 et 42 jours d'application biquotidienne du principe actif selon l'invention ou du placebo sur l'ensemble du visage.

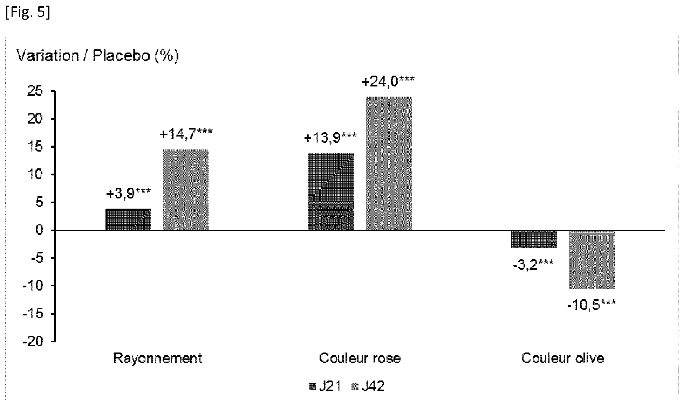

Les résultats correspondant à l'effet du principe actif selon l'invention formulé à 3% en émulsion sur l'éclat du teint pour le panel caucasien et asiatique sont respectivement présentés sur les figures 4 et 5.

La figue 4 montre que le principe actif selon l'invention améliore significativement les paramètres caractéristiques de l'éclat du teint de volontaires caucasiens. En effet, dès 21 jours d'applications, le principe actif selon l'invention formulé à 3% en émulsion rend le teint plus lumineux et plus frais en augmentant significativement le rayonnement de la peau de 7,8% et la couleur rose de 21,2%. Il diminue significativement la couleur olive de 13,2% ainsi que l'état de fatigue des yeux de 11,1%. Ces actions se prolongent après 42 jours de traitement avec une augmentation de 12,5% du rayonnement de la peau et de 23,1% de la couleur rose. On observe également une diminution de la couleur olive de 13,0% et de l'état de fatigue des yeux de 14,6% chez les volontaires caucasiens.

La figure 5 montre que le principe actif selon l'invention améliore significativement les paramètres caractéristiques de l'éclat du teint de volontaires asiatiques. En effet, dès 21 jours d'applications, le principe actif selon l'invention formulé à 3% en émulsion rend le teint plus lumineux et plus frais en augmentant significativement le rayonnement de la peau de 3,9% et la couleur rose de 13,9%. Il diminue significativement la couleur olive de 3,2%. Ces actions se prolongent après 42 jours de traitement avec une augmentation de 14,7% du rayonnement de la peau et de 24% de la couleur rose. On observe également une diminution de la couleur olive de 10,5% chez les volontaires asiatiques.

Ainsi, le principe actif selon l'invention formulé à 3% en émulsion, améliore l'éclat du teint de volontaires caucasiens et asiatiques.

## Revendications

1. Utilisation cosmétique d'un principe actif cosmétique comprenant au moins un extrait de *Spirodela polyrhiza* comprenant des apiogalacturonanes, pour un effet repulpant.

2. Utilisation selon la revendication précédente, **caractérisée en ce que** le principe actif présente également un effet hydratant et/ou améliore l'éclat du teint.

3. Utilisation selon l'une des revendications précédentes, **caractérisée en ce que** le principe actif augmente le volume cellulaire cutané, et/ou l'expression de l'aquaporine 3 et/ou la synthèse du transporteur BGT-1.

4. Principe actif cosmétique comprenant au moins un extrait de *Spirodela polyrhiza,* **caractérisé en ce que** l'extrait est un hydrolysat et comprend des apiogalacturonanes.

5. Principe actif selon la revendication précédente, **caractérisé en ce que** l'extrait présente un potentiel hygroscopique supérieur ou égal à 15 molécules d'eau en interaction par monomère.

6. Principe actif selon l'une des revendications 4 ou 5, **caractérisé en ce que** les apiogalacturonanes représentent au moins 40% en poids de la matière sèche de l'extrait.

7. Principe actif selon l'une des revendications 4 à 6, **caractérisé en ce que** l'extrait comprend des carbohydrates représentant au moins 70% en poids de la matière sèche de l'extrait.

8. Principe actif selon l'une des revendications 4 à 7, **caractérisé en ce que** l'extrait présente un ratio d'acide galacturonique / apiose compris entre 1 et 5.

9. Principe actif selon l'une des revendications 4 à 8, **caractérisé en ce que** l'extrait est susceptible d'être obtenu par un procédé comprenant les étapes suivantes :
a. Solubilisation d'au moins 50 g/L de *Spirodela polyrhiza* dans l'eau,
b. Hydrolyse chimique de nature acide, ou hydrolyse chimique de nature acide et hydrolyse enzymatique,
c. Séparation des phases solubles et insolubles et récupération de la phase soluble,
d. Purification et concentration de la phase soluble,
e. Filtration et filtration stérilisante.

10. Composition cosmétique pour une application topique comprenant au moins un principe actif selon l'une des revendications 4 à 9 et un milieu physiologiquement acceptable.

11. Composition cosmétique selon la revendication précédente, dans laquelle le principe actif représente au moins 0,1% en poids du poids total de la composition.

12. Utilisation cosmétique selon l'une des revendications 1 à 3 **caractérisé en ce que** le principe actif est selon l'une des revendications 4 à 9 ou la composition cosmétique est selon l'une des revendications 10 ou 11.
